# EUROPEAN PATENT SPECIFICATION

(11) **EP 0 656 118 B1**
(45) Date of publication and mention of the grant of the patent: **12.02.1997**
(21) Application number: 93917783.8
(22) Date of filing: 10.08.1993
(51) Int. Cl.: G01N 33/74, G01N 33/76

(54) **MONITORING METHOD**
ÜBERWACHUNGSVERFAHREN
PROCEDE DE CONTROLE DE CYCLE D'OVULATION

(30) Priority: 21.08.1992 GB 9217866
(43) Date of publication of application: 07.06.1995
(60) Divisional application: 96112234.8
(73) Proprietor: UNIPATH LIMITED, Basingstoke, Hampshire RG24 OPW (GB)
(72) Inventor: CATT, Michael 14 Brampton Close, Northampton NN8 5XG (GB); COLEY, John 10 The Avenue, Northampton NN9 6PT (GB); DAVIS, Paul, James The Hawthorns, Felmersham Bedford MK43 7EX (GB)
(74) Representative: Butler, David John
(86) International application number: EP9302146
(87) International publication number: WO9404924

(56) References cited:
- EP-A- 0 291 194
- EP-A- 0 367 615
- CONTRACEPTION vol. 33, no. 4 , April 1986 , LOS ALTOS, CA, US pages 327 - 345 S.Z. CEKAN ET AL. 'The Prediction and/or Detection of Ovulation by Means of Urinary Steroid Assays'
- HUMAN REPRODUCTION vol. 6, no. 4 , April 1991 , OXFORD, GB pages 515 - 518 P. BISCHOF, P.G. BIANCHI AND A. CAMPANA 'Comparison of a rapid, quantitative and automated assay for urinary luteinizing hormone (LH), with an LH detection test, for the prediction of ovulation'
- INTERNATIONAL JOURNAL OF GYNECOLOGY AND OBSTETRICS vol. SUPPL., no. 1 , 1989 , SHANNON, IE pages 111 - 122 J.B. BROWN, L.F. BLACKWELL, J. HOLMES AND K. SMYTH 'New assays for identifying the fertile period' cited in the application
- INTERNATIONAL JOURNAL OF GYNECOLOGY AND OBSTETRICS vol. SUPPL., no. 1 , 1989 , SHANNON, IE pages 35 - 43 W.P. COLLINS 'Biochemical indices of potential fertility'
- FERTILITY AND STERILITY vol. 47, no. 2 , February 1987 , BIRMINGHAM, ALA., US pages 259 - 264 MICHAEL VERMESH ET AL. 'Monitoring techniques to predict and detect ovulation'
- FERTILITY AND STERILITY vol. 44, no. 3 , September 1985 , BIRMINGHAM, ALA., US pages 328 - 334 LARS E. M. SCHIPHORST, WILLIAM P. COLLINS, J. PATRICK ROYSTON 'An estrogen test to determine the times of potential fertility in women'
- STEROIDS vol. 31, no. 2 , February 1978 , SAN FRANCISCO US pages 175 - 187 S.M. JUDGE ET AL. 'Time-course relationships between serum LH, serum progesterone and urinary pregnanediol concentration in normal women'
- INTERNATIONAL JOUNAL OF FERTILITY vol. 30, no. 3 , March 1985 , LAWREENCE, KANSAS, US pages 18 - 30 WORLD HEALTH ORGANISATION, GENEVA, CH 'A Prospective Multicentre Study to Develop Universal Immunochemical Tests for Predicting the Fertile Period in Women'
- SCIENCE vol. 248 , 1 June 1990 , LANCASTER, PA US pages 1061 - 1062 CARL DJERASSI 'Fertility Awareness: Jet-Age Rhythm Method?'

## Description

This invention relates to methods, devices and test kits for use in monitoring the ovulation cycle in female mammals especially humans.

The invention is particularly, although not solely concerned with the provision of reliable information concerning fertility status as an aid to contraception, by the use of simple practical procedures that can readily be applied by unskilled persons e.g. in the home. An important objective of the invention is to provide reliable contraceptive advice while avoiding the necessity for tests to be conducted on a frequent (eg. daily) basis throughout every ovulation cycle. The necessity for regular, e.g. daily, testing throughout the cycle has characterised many ovulation cycle monitoring systems previously proposed.

To provide reliable information concerning fertility status, the user must be given adequate warning of the onset of the fertile phase in the cycle. In general the proposed techniques rely on the monitoring of one or more parameters which alter as the event of ovulation approaches. Typical parameters which have been invoked are the concentration of a body fluid analyte, such as estradiol and metabolites thereof, for example estrone-3-glucuronide (E3G). Other parameters that have been used are basal body temperature and various physiological changes such as the characteristics of vaginal mucous.

Many excellent academic studies have been carried out using such parameters. Such studies have established how these parameters can be correlated with the fertility status of an average member of a large population sample.

However, when attempting to develop a practical monitoring system suitable for use by individuals, it is found that many individual subjects do not conform to the average in terms of cycle length and/or the duration and timing of the fertile phase. The extent of variation from one individual to another, and indeed, from one cycle to another in the same individual, renders average population data too unreliable for practical use.

In the provision of reliable contraceptive advice, it is clearly very important that a monitoring method should give adequate warning of the onset of a fertile phase which happens to occur significantly in advance of normal. In this instance "normal" may be defined either in terms of population data, or normal for the individual subject herself.

The tendency has been to exercise extreme caution and to require testing of the relevant parameters throughout the cycle, and particularly right from the onset of the cycle (onset of menses). From the individual user's point of view, it is clearly advantageous if the necessity for such constant testing could be avoided and, instead, for the testing to be performed over a comparatively brief portion of each cycle. Not merely may this benefit the user in terms of convenience, but the cost of the method may also be reduced if, for example, fewer disposable testing devices are required each month.

In principle, based on population information, it should be possible to reduce the number of tests required by deciding that tests are unnecessary until a universally defined numerical day, or other specified time interval, following the onset of menses. However, as indicated above, individual variation tends to make any such universal assumption unsafe.

Cekan et al, Contraception, Vol 33 No. 4 (1991) p327-45 compares a range of urinary analytes of possible use in the prediction of ovulation or fertile period, in experiments involving the collection of daily urine samples during a complete cycle. Cekan et al conclude that although for the prediction of ovulation or fertile period, the best index of urinary steroids appears to be the sustained rise in the ratio of E1G to C, this "best" method is still not good enough in terms of overall reliability and practicability.

Bischof et al, Human Reproduction, Vol.6 No.4 (1991) p 515-18 is an academic study to evaluate a rapid automated assay for LH.

Brown et al, Int.J.Gynecol.Obstet. (1989) Suppl.1 p.111-22 describes a method for identifying the fertile period involving assays of urinary analytes. Testing is daily and started as soon as menstruation ceases or earlier.

Collins, Int.J.Gynecol.Obstet. (1989) Suppl.1, p.35-43 compares various biochemical indices of potential fertility, but does not propose any new approach involving minimising the number of tests required.

EP-A-291194 discloses assay devices that can be used to measure urinary analytes of relevance to fertility, but does not propose any particular testing regime.

For the purposes of this specification, estradiol and all measurable estradiol metabolites, will collectively be referred to henceforth as "E3G". In addition to estrone-3-glucuronide already mentioned, estradiol metabolites that can also be assayed for the purposes of the invention include estradiol-3-glucuronide, estradiol-17-glucuronide, estriol-3-glucuronide, estriol-16-glucuronide and (principally for non-human subjects) estrone-3-sulphate.As will be appreciataed from the following description, the invention can readily be applied to data derived from the measurement of body fluid concentrations of other analytes of significance in relation to the status of the ovulation cycle. Generally, the most suitable analytes are hormones and their metabolites. Follicle stimulating hormone (FSH) is an example. Examples of alternative body fluids, which are relatively accessible, are saliva, crevicular fluid, sweat, sebum, tears and vaginal fluid. In principle internal fluids, such as blood, can be used but are generally not preferred because they can only be accessed readily by invasive techniques.

The skilled reader will also appreciate that the body fluid "concentration" of the chosen analyte or analytes need not be measured in absolute terms, although this can of course be done if desired. Generally, it will be sufficient to assay an analyte in a manner which yields a signal, convertible to numerical data, related to the actual concentration, so that such data can be compared with similar data obtained at a different stage in the cycle to determine whether or not a significant change in actual concentration has occurred. Accordingly, where the specification and claims below refer to the "concentration" of an analyte, this expression should be interpreted broadly.

The invention provides a method of monitoring the status of a current ovulation cycle of an individual mammalian, eg human, female subject, involving repeated (i.e. regular, e.g. daily) testing of the body fluid concentration of at least one analyte of significance in relation to the status of the ovulation cycle during at least the pre-ovulation phase of the current ovulation cycle of the individual subject, wherein testing for said analyte concentration during the current ovulation cycle is commenced a plurality (preferably at least 5) days following the onset of menses but at least 2 numerical days in advance of the earliest numerical day on which actual ovulation has occurred in one or more previous ovulation cycles in the same individual subject.

Preferably, testing is commenced at least 3, and more preferably at least 4, numerical days in advance of the earliest numerical day on which actual ovulation has occurred in one or more previous ovulation cycles in the same individual subject.

Conveniently, the body fluid can be urine. A very suitable analyte is estradiol or a metabolite thereof, such as E3G.

In one embodiment of the invention, 2 or more analytes are tested to provide comparative data, such as the concentration ratio of two analytes tested simultaneously.

Preferably, the earliest actual ovulation day is derived from data collected during at least 3, and more preferably at least 5, consecutive previous cycles.

Ideally, the earliest ovulation day used to calculate the time interval for the purposes of the current cycle is derived from data obtained during at least the immediately preceding cycle.

A particularly convenient method involves the determination of the earliest previous ovulation day from data obtained from a "rolling" reference base consisting of a fixed number of consecutive cycles immediately preceding the current cycle. Preferably this rolling reference base consists of the immediately preceding 4 to 10 cycles, more preferably the immediately preceding 5 or 6 cycles. By having such a rolling reference base, any progressive "drift" in the occurrence of ovulation in the individual concerned can be picked up and accounted for in the allocation of the next testing commencement day.

The invention includes a test kit comprising one or more testing devices for determining the concentration (in relative or absolute terms) of said at least one analyte in said body fluid, together with instructions advising the user to commence said testing during said time interval, and means enabling a user to derive said time interval and/or a precise testing commencement day from knowledge of the numerical day on which actual ovulation occurred during at least one previous ovulation cycle.

More generally the invention can be applied in any method of monitoring the status of a current ovulation cycle of an individual mammalian, eg human, female subject involving the measurement of a parameter of significance in relation to the status of ovulation cycle and which exhibits a detectable change during the pre-ovulation phase of the cycle occurring at least 2 and more preferably at least 3 days in advance of the day of actual ovulation.

In a preferred embodiment of the invention, the choice of day for commencement of testing is influenced by the time interval between the onset of menses and the earliest numerical day on which actual ovulation has previously occurred. In some individuals there can be a comparatively long interval between the onset of menses and the first occurrence of ovulation. This is commonly observed in human individuals having cycle lengths which are frequently or always longer than the "28 day" population average. In such individuals there is a considerable risk that occasionally ovulation will occur exceptionally early (for that individual) and the testing commencement day should be brought forward to allow for this. Preferably the testing commencement day is calculated according to the following relationship:

| Earliest previous ovulation day | Testing commencement day |
|---|---|
| 8 - 10 | -4 |
| 11 - 14 | -5 |
| 15 - 18 | -6 |
| 19 - 23 | -7 |
| 24 - 28 | -8 |
| 29+ | -9 |

The parameter(s) chosen for providing the warning of imminent ovulation are not critical to the invention, provided that the parameter exhibits a detectable change within the time interval between the commencement of testing (as determined herein) and a safe time in advance of actual ovulation in the current cycle.

In general, however, the most suitable parameter will be a change in the concentration (absolute or relative) of an analyte in a body fluid, especially a hormone or a metabolite thereof. The most appropriate body fluid is usually urine.

In an article by Brown et al, Int. J. Gynecol. Obstet. 1989 Suppl. 1, pages 111-122, entitled "New assays for identifying the fertile period", a method is described in which urinary estrogen is measured, and the statement is made that:
".... the daily increment in estradiol production during the rise to the pre-ovulatory peak is remarkably constant for all cycles, being very close to a factor of 1.4 per day."

The present invention can be applied usefully in any method of monitoring the ovulation cycle which involves incremental ("ratiometric") analysis of a detectable variable parameter of significance during the pre-ovulatory phase of the cycle and wherein a warning of imminent ovulation is given when a pre-determined reference ratio is identified. Indeed, the present invention can be applied to advantage in any assay system which involve mathematical analysis of successive analyte concentrations, particularly urinary E3G concentrations by, for example, "CUSUM" analysis. Usually, the successive analyte concentrations (or indeed, successive measurements of any relevant parameter in accordance with the invention) are recorded on successive days.

The following description is provided, by way of example only, in relation to the particular urinary hormones E3G, lutenising hormone (LH), and pregnanediol-3-glucuronide (P3G), although it will be readily appreciated that the principles of the method can be used in relation to other biochemical markers, for example the hormones estradiol and progesterone, found for example in the blood or in saliva. The method of the invention may be applied to, or used in combination with observations of other physiological signs of the level of fertility in a female, of which she is aware, or can readily be made aware of, e.g. markers in other body fluids.

Ovulation day can be determined by any of the known chemical or physiological parameters, although a preferred method is by measuring the level of LH. Once the LH surge has been detected, it can be said that ovulation of the user is imminent. Also, the day of the cycle on which ovulation has occurred can be noted for future reference. If the LH surge is detected, and hence the day of ovulation accurately pinpointed, it can be indicated to the user with a very high degree of certainty that the user will no longer be fertile four days hence (3 days after ovulation). For practical purposes, a urinary LH concentration of 20 mIU/ml can be regarded as a universal threshold indicative of the LH surge under virtually all circumstances.

LH surge detection is most preferred for this purpose. The expression "LH surge" is used herein to mean the dramatic rise in LH concentration that precedes the event of ovulation. In the art, reference is made also to "LH max", i.e. the peak concentration of LH. In the majority of individuals, these are for all practical purposes simultaneous, when the cycle is monitored on a day-by-day basis. However, in a few individuals, perhaps 20% of the population, the actual peak concentration of LH is not observed until the day following the main concentration rise. For the purposes of the invention, we prefer to use the observable rise as the critical parameter.

A method for predicting the end of the fertile period (though not so accurately the day of ovulation) is to measure the levels of the urinary hormone P3G. P3G has a relatively low level in urine until the start of the luteal phase, at which point its level rises fairly sharply. Therefore, once an elevated level of P3G is detected, it can be indicated to the user that the luteal phase of the cycle - ie. the terminal infertile period - has commenced. An elevated level of urinary P3G can be based on data taken during the current and/or one or more preceding cycles. An "elevated" P3G level can be recorded, for example, when either the level of P3G detected is greater than the sum of the four previous recorded levels of P3G in the same menstrual cycle, or greater than 3500 ng/ml, whichever of these two thresholds is lower and is first achieved. Once an "elevated" P3G level is recorded, the user can be that she is infertile for the remainder of that cycle.

In a preferred embodiment, the detection of either LH or P3G can be used as a trigger to indicate to the user that the user is no longer fertile until the end of the cycle, with one hormone acting as a "back up" to the other. However, it is preferred that the detection of LH be used as a primary indicator of whether ovulation has or is about to occur, since the detection of LH lends itself to more accurate determination of the exact ovulation day than the use of P3G.

Methods of detecting body fluid analytes, such as urinary hormone metabolites, suitable for the purposes of this method, are well known to those skilled in the art. In a preferred embodiment, the analyte is detected by assay methods and devices as described in GB-A-2204398.

Where the method of the invention relies on measurement of a urine component, this must be done on a urine sample. A variety of immunoassay techniques are available which enable urine components to be measured. A wide variety of solid phase testing devices such as dipsticks and chromatographic strips have been described in the literature, and can readily be adapted for use in determining urinary analytes. The device should at least be capable of indicating relative levels of analyte, eg. E3G, in threshold bands. Examples of simple assay technology that can readily be adapted for use in the home is described, for example, in EP-A-0225054, EP-A-0183442, EP-A-0186799, GB-A-2204398 and EP-A-383619. Disposable assay strips such as those described in GB-A-2204398 and EP-A-383619 which simply require to be contacted with urine and which provide an assay result in semi-qualitative form, eg. by means of a series of test zones on the strip which are progressively positive at higher urinary analyte levels, can be used. Multiple strips that respond at different analyte thresholds can be used, rather than a single strip. Alternatively, a visually readable quantitative assay can be based on progression of a visible, eg. coloured, region or "front" over a surface (eg. radial diffusion), using for example an enzyme-labelled assay.

In a more sophisticated version of an apparatus according to the invention, the recording device can incorporate means for reading the result of the urine assay, eg. by measuring the reflectance or fluorescence from an assay strip. This may enable a more precise numerical indication to be given of the analyte level, and further enhance the accuracy of the method.

In an embodiment of the invention in which two or more analytes are measured simultaneously, such measurement can if desired be performed using a single body fluid testing device, eg. a device incorporating multiple assay strips, or a single strip capable of independently detecting the level of the different analytes.

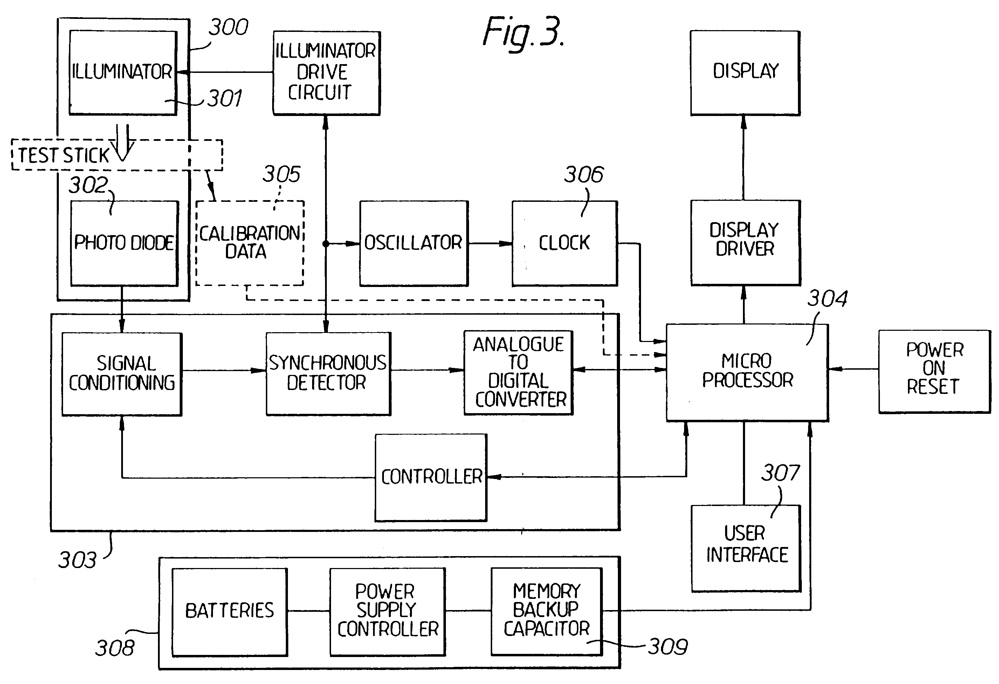

The detailed electronics of a recording device capable of assimilating, remembering and handling analyte concentration data, as well as providing the preferred electronic features of the device discussed herein, and predicting future cycles on the basis of such data, can readily be provided by those skilled in the electronics art once they have been advised of the factors that such a device must take into consideration, and the information that the device must provide for the user. Such detailed electronics do not form part of the invention. However, by way of example only, the basic functions that may be required in such a device are outlined in Figure 3 of the accompanying drawings and described briefly below.

The invention includes an eletronic means programmed for use in a method of the invention.

By way of example only, practical aspects of the invention are described below with reference to the accompanying drawings, of which:

Figure 1 of the accompanying drawings illustrates an ovulation cycle monitoring device for use in accordance with the invention, together with an associated urine sample testing device.

Figure 2 shows the urine testing device in greater detail.

Figure 3 shows, in schematic form, the basic functions that may be required in an electronic monitor for use in accordance with the invention.

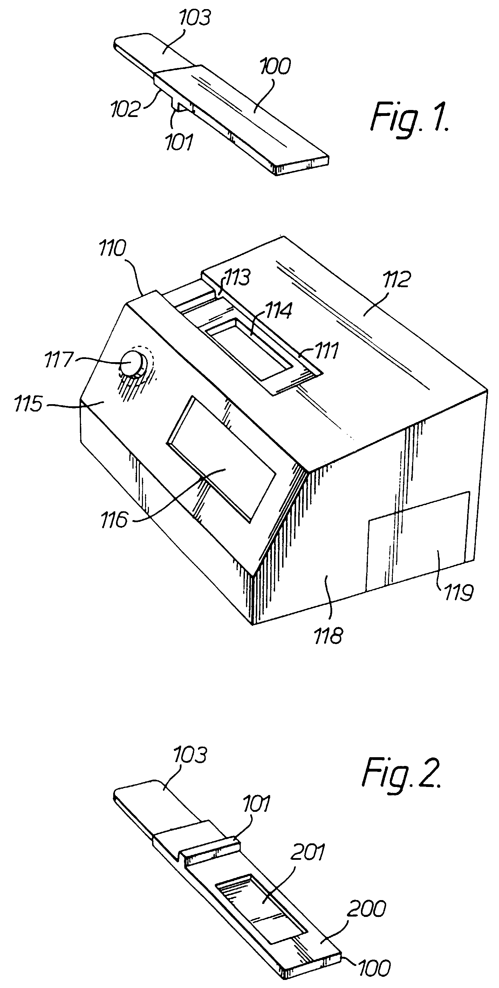

Referring to Figure 1, the urine sample testing device comprises a flat elongate casing 100 having a locating ridge 101 on its lower surface 102. Projecting from one end of the casing is a bibulous sample receiving member 103.

The monitor comprises a casing 110 having a recess 111 in its upper surface 112 to accommodate the casing 100 of the testing device. Recess 111 incorporates a locating slot 113 into which the locating ridge 101 on the casing of the testing device can be inserted to positively locate the testing device in relation to a reading window 114 in the recess. Casing 110 contains means (not shown) such as a fluorescence reader to measure the result of a urinary analyte concentration assay performed using the testing device.

The sloping front face 115 of the monitor casing incorporates a large window 116 through which information can be conveyed to the user eg. by means of an LED display or other visual output. This information can be provided in a variety of forms, such as an indication of a calender and the need to perform urine tests, and an indication of the current status of the ovulation cycle. The sloping face 115 of the casing also incorporates a button 117 which the user can press to indicate the commencement of an ovulation cycle and to start the monitor processing information relative to that cycle.

Information can be conveyed to the user by means of a liquid crystal or LED display, for example. If desired, information on the state of fertility can be conveyed by a simple visual indication, eg a combination of colours showing, for example, green for infertile, red for fertile, and yellow for any intermediate stage when conception is less likely but still possible. Conveniently, the red and yellow signals on the device may be combined, such that the device indicates "red" signal to the user whenever conception is possible. Especially if the device is intended primarily as an aid to contraception, it should "fail safe" by showing a "fertile" signal.

The invention further provides a kit for monitoring the ovulation cycle of a female mammal, comprising a monitoring device as set forth above, together with at least one testing device capable of being used to measure the level of one or more urine components. It is envisaged that the monitoring device will generally be of a relatively durable nature and capable of being used over a considerable number of cycles. The testing devices for measuring the urine components are preferably disposable after individual use, and it is therefore envisaged that the user of the monitoring device will need to replenish the testing devices.

An embodiment of the invention is a plurality of disposable body fluid (eg urine) testing devices, packaged with instruction for use in a method according to the invention. These testing devices, where appropriate, can be urine testing devices from each of which the urinary concentrations of E3G and LH can be determined (where necessary, in conjunction with a monitor device or electronic means as set forth herein).

In general the monitor will be battery-powered, and incorporates in side 118 of the casing an access point such as a removable cover 119 to permit batteries to be inserted and changed.

Referring to Figure 2, the testing device is shown inverted relative to the aspect seen in Figure 1. The locating ridge 101 is now on the upper surface 200. Also in the surface 200 now uppermost is a result window 201. The body of the testing device can incorporate an immunochromatograhic strip (not shown) incorporating all necessary reagents to enable an immunoassay to be formed which detects the presence and concentration of analyte in a urine sample applied to the sample collecting member 103. The result of the assay can be effected by the immobilization of a labelled component, via a sandwich or competition reaction in the presence of analyte in an applied urine sample, the labelled reagent becoming concentrated in a zone revealed through the result window. When the testing device is inverted and located in the recess 111 in the casing of the monitor, the result window is immediately adjacent to the reading window 114 in the monitor and the assay result can be determined. For example, if the label is a fluorescent reagent, the reading means within the monitor can detect and measure fluorescent light output from the accumulated label in the detection zone on the strip to provide a numerically accurate concentration value for the analyte in the urine sample. This information can be processed by the monitor together with calender information resulting from the initiation of the cycle process by the user and historical data which the monitor can retain from previous cycles.

Referring to Figure 3, some of the basic elements which may be required in an electronic monitoring device are seen. The individual features can be entirely conventional, and those familiar with the art of electronics will appreciate that other combinations and arrangements of such features can be employed to achieve the objectives of the invention. For example, so-called "hard-wired" systems, and "neural networks", can be used in place of conventional microprocessors based on "chip" technology. As depicted in Figure 3, the combination essentially comprises:

A reading unit 300 to derive information from a test device, such as a test stick, the reading unit comprising an illuminator 301 and a reader 302 (represented here as a photo diode). The reading unit feeds into a conversion unit 303 to convert the optical signal into a form usable by a microprocessor 304. As an optional feature, a calibration system 305 is provided to convert the signal derived from the reading unit into data corresponding, for example, to an absolute concentration value. A timer, such as a clock 306 is required to regulate measurements within a cycle. The microprocessor 304 processes, memorizes and interprets results in the light of previous events, particularly recorded from previous cycles. The user interface 307 will generally comprise at least means, such as a push button, which the user can operate at the commencement of a cycle to initiate the operation of the device as a whole. The power supply 308 should include means, such as a memory back up capacitator 309, to prevent loss of historical data if it becomes necessary to replace batteries.

Aspects of the invention are illustrated in the following Examples. These relate to the monitoring of the human ovulation cycle.

The example shows E3G profiles from two women - one known to have low levels of urinary E3G and the other known to have relatively high levels. In the first two columns of each table, 30 days of each cycle are set out in terms of their fertility. The first phase is termed infertile and consists of that portion of the follicular phase during which unprotected intercourse would not be expected to result in conception, followed by a transitional phase during which changes occur that lead to a fertile state and during which a positive signal to indicate the onset of the fertile phase is required. The fertile phase is that phase before and after ovulation during which unprotected intercourse is most likely to result in conception. Its duration before ovulation is dictated entirely by the effective lifetime of sperm, and this, in turn is influenced by factors controlled by the female hormones, especially mucus. The post fertile, luteal phase is that time after which the ovum has left the uterus and conception in the current cycle is no longer possible.

E3G values for the first 20 days of each cycle are given in the third column. These were derived by immunoassay on early morning urine samples collected each day. The immunoassay was a conventional enzyme-labelled-antigen competitive assay. The values given are in ng/ml.

Actual ovulation is taken as 24 hours following the LH maximum value. These LH values were determined by conventional immunoassay on the same samples, but the values are not included in the table as ovulation date is the essential result.

Following the initial cycle in each individual, a testing commencement day applicable in a home-use situation was chosen for the next cycle in accordance with the tabulated relationship given earlier in this specification. Thus, for example, following cycle Al in which actual ovulation occurred on numerical day 18, the chosen testing commencement day for cycle A2 was numerical day 12 (18 - 6).

Examining cycle A2 it can be seen that, in practice, commencing testing on day 12 would have coincided with the beginning of the E3G rise indicative of imminent ovulation. Actual ovulation occurred on day 17. Applying the tabulated relationship the testing commencement day chosen for cycle A3 was numerical day 11.

In practice in cycle A3 numerical day 11 was merely 4 days in advance of actual ovulation which occurred on day 15. The testing commencement day chosen for cycle A4 was day 9.

It can be seen from cycle A4 that day 9 was 5 days in advance of actual ovulation (on day 14) and that the recommended testing commencement day for the following cycle was also day 9.

In cycle A5, actual ovulation occurred on day 17 and probably the drift of actual ovulation towards earlier days in successive cycles in this individual had ceased, and actual ovulation is reverting to a more "normal" timing. The recommended testing commencement day should however remain as numerical day 9, at least for the time being, to cope with another early drift of ovulation day.

This series of cycles shows the advantage of using a succession of consecutive cycles to establish the optimum testing commencement day.

For individual B the testing commencement day recommended from cycle B1 was numerical day 13, which was modified after cycle B2 to numerical day 11, and after cycle B4 to day 10.

### INDIVIDUAL A

| CYCLE A 1: Profile-establishing cycle | | | |
|---|---|---|---|
| Day | Phase | E3G value | Actual Ovulation |
| 1 | infertile | 2.7 | |
| 2 | " | 3.3 | |
| 3 | " | 2.4 | |
| 4 | " | 1.8 | |
| 5 | " | 3.6 | |
| 6 | " | 2.5 | |
| 7 | " | 1.7 | |
| 8 | " | 1.9 | |
| 9 | " | 3.1 | |
| 10 | " | 5.4 | |
| 11 | " | 2.1 | |
| 12 | " | 5.3 | |
| 13 | " | 10.5 | |
| 14 | " | 7.7 | |
| 15 | fertile | 5.2 | |
| 16 | " | 8.3 | |
| 17 | " | 6.8 | |
| 18 | " | 4.3 | LHM + 1 |
| 19 | " | 4.9 | |
| 20 | " | 5.3 | |
| 21 | postfertile | | |
| 22 | " | | |
| 23 | " | | |
| 24 | " | | |
| 25 | " | | |
| 26 | " | | |
| 27 | " | | |
| 28 | " | | |
| 29 | " | | |
| 30 | " | | |

Chosen testing commencement day for next cycle: 12

| CYCLE A 2 | | | |
|---|---|---|---|
| Day | Phase | E3G value | Actual Ovulation |
| 1 | infertile | 2.0 | |
| 2 | " | 1.5 | |
| 3 | " | 1.1 | |
| 4 | " | 4.1 | |
| 5 | " | 4.0 | |
| 6 | " | 3.5 | |
| 7 | " | 2.3 | |
| 8 | " | 1.9 | |
| 9 | " | 3.6 | |
| 10 | " | 3.7 | |
| 11 | " | 3.0 | |
| 12*** | " | 9.2 | |
| 13 | " | 8.9 | |
| 14 | fertile | 14.6 | |
| 15 | " | 12.6 | |
| 16 | " | 8.8 | |
| 17 | " | 15.8 | LHM + 1 |
| 18 | " | 6.9 | |
| 19 | " | 6.5 | |
| 20 | postfertile | 6.5 | |
| 21 | " | | |
| 22 | " | | |
| 23 | " | | |
| 24 | " | | |
| 25 | " | | |
| 26 | " | | |
| 27 | " | | |
| 28 | " | | |
| 29 | " | | |
| 30 | " | | |

Days in advance of actual ovulation: 5
Chosen testing commencement day for next cycle: 11

| CYCLE A 3 | | | |
|---|---|---|---|
| Day | Phase | E3G value | Actual Ovulation |
| 1 | infertile | 2.0 | |
| 2 | " | 2.0 | |
| 3 | " | 2.7 | |
| 4 | " | 2.9 | |
| 5 | " | 2.7 | |
| 6 | " | 1.6 | |
| 7 | " | 2.5 | |
| 8 | " | 5.4 | |
| 9 | " | 4.0 | |
| 10 | " | 5.6 | |
| 11*** | " | 3.7 | |
| 12 | fertile | 6.2 | |
| 13 | " | 23.6 | |
| 14 | " | 21.3 | |
| 15 | " | 8.3 | LHM + 1 |
| 16 | " | 4.5 | |
| 17 | " | 3.7 | |
| 18 | postfertile | 3.4 | |
| 19 | " | 2.8 | |
| 20 | " | 3.1 | |
| 21 | " | | |
| 22 | " | | |
| 23 | " | | |
| 24 | " | | |
| 25 | " | | |
| 26 | " | | |
| 27 | " | | |
| 28 | " | | |
| 29 | " | | |
| 30 | " | | |

Days in advance of actual ovulation: 4
Chosen testing commencement day for next cycle: 9

| CYCLE A 4 | | | |
|---|---|---|---|
| Day | Phase | E3G value | Actual Ovulation |
| 1 | infertile | 2.4 | |
| 2 | " | 2.8 | |
| 3 | " | 5.2 | |
| 4 | " | 3.6 | |
| 5 | " | 2.5 | |
| 6 | " | 3.1 | |
| 7 | " | 3.9 | |
| 8 | " | 4.6 | |
| 9*** | " | 5.1 | |
| 10 | " | 6.1 | |
| 11 | fertile | 16.7 | |
| 12 | " | 10.8 | |
| 13 | " | 22.8 | |
| 14 | " | 21.3 | LHM + 1 |
| 15 | " | 9.4 | |
| 16 | " | 12.2 | |
| 17 | postfertile | 5.7 | |
| 18 | " | 5.2 | |
| 19 | " | 7.5 | |
| 20 | " | 9.0 | |
| 21 | " | | |
| 22 | " | | |
| 23 | " | | |
| 24 | " | | |
| 25 | " | | |
| 26 | " | | |
| 27 | " | | |
| 28 | " | | |
| 29 | " | | |
| 30 | " | | |

Days in advance of actual ovulation: 5
Chosen testing commencement day for next cycle: 9

| CYCLE A 5 | | | |
|---|---|---|---|
| Day | Phase | E3G value | Actual Ovulation |
| 1 | infertile | 2.3 | |
| 2 | " | 2.8 | |
| 3 | " | 2.7 | |
| 4 | " | 2.3 | |
| 5 | " | 2.8 | |
| 6 | " | 4.8 | |
| 7 | " | 5.6 | |
| 8 | " | 4.5 | |
| 9*** | " | 3.2 | |
| 10 | " | 8.5 | |
| 11 | " | 7.3 | |
| 12 | " | 6.3 | |
| 13 | " | 7.0 | |
| 14 | fertile | 11.8 | |
| 15 | " | 19.3 | |
| 16 | " | 18.5 | |
| 17 | " | 9.2 | LHM + 1 |
| 18 | " | 5.3 | |
| 19 | " | 4.8 | |
| 20 | postfertile | 6.1 | |
| 21 | " | | |
| 22 | " | | |
| 23 | " | | |
| 24 | " | | |
| 25 | " | | |
| 26 | " | | |
| 27 | " | | |
| 28 | " | | |
| 29 | " | | |
| 30 | " | | |

Days in advance of actual ovulation: 8
Chosen testing commencement day for next cycle: 9

### INDIVIDUAL B

| CYCLE B1: Profile-establishing cycle | | | |
|---|---|---|---|
| Day | Phase | E3G value | Actual Ovulation |
| 1 | infertile | 10.9 | |
| 2 | " | 15.2 | |
| 3 | " | 21.2 | |
| 4 | " | 12.7 | |
| 5 | " | 11.8 | |
| 6 | " | 16.5 | |
| 7 | " | 15.6 | |
| 8 | " | 25.1 | |
| 9 | " | 10.1 | |
| 10 | " | 16.8 | |
| 11 | " | 28.2 | |
| 12 | " | 24.6 | |
| 13 | " | 28.7 | |
| 14 | " | 27.7 | |
| 15 | " | 62.6 | |
| 16 | " | 68.5 | |
| 17 | fertile | 61.9 | |
| 18 | " | 103.4 | |
| 19 | " | 85.4 | |
| 20 | " | 45.4 | LHM + 1 |
| 21 | " | | |
| 22 | " | | |
| 23 | postfertile | | |
| 24 | " | | |
| 25 | " | | |
| 26 | " | | |
| 27 | " | | |
| 28 | " | | |
| 29 | " | | |
| 30 | " | | |

Chosen testing commencement day for next cycle: 13

| CYCLE B 2 | | | |
|---|---|---|---|
| Day | Phase | E3G value | Actual Ovulation |
| 1 | infertile | 27.5 | |
| 2 | " | 28.8 | |
| 3 | " | 24.7 | |
| 4 | " | 22.6 | |
| 5 | " | 24.9 | |
| 6 | " | 28.9 | |
| 7 | " | 14.6 | |
| 8 | " | 8.4 | |
| 9 | " | 24.7 | |
| 10 | " | 33.6 | |
| 11 | " | 39.3 | |
| 12 | " | 25.6 | |
| 13*** | " | 43.2 | |
| 14 | " | 67.1 | |
| 15 | fertile | 62.0 | |
| 16 | " | 94.6 | |
| 17 | " | 58.4 | LHM + 1 |
| 18 | " | 42.4 | |
| 19 | " | 60.4 | |
| 20 | " | 56.0 | |
| 21 | postfertile | | |
| 22 | " | | |
| 23 | " | | |
| 24 | " | | |
| 25 | " | | |
| 26 | " | | |
| 27 | " | | |
| 28 | " | | |
| 29 | " | | |
| 30 | " | | |

Days in advance of actual ovulation: 4
Chosen testing commencement day for next cycle: 11

| CYCLE B 3 | | | |
|---|---|---|---|
| Day | Phase | E3G value | Actual Ovulation |
| 1 | infertile | 46.4 | |
| 2 | " | 30.0 | |
| 3 | " | 12.4 | |
| 4 | " | 6.5 | |
| 5 | " | 8.7 | |
| 6 | " | 17.2 | |
| 7 | " | 14.9 | |
| 8 | " | 11.8 | |
| 9 | " | 11.0 | |
| 10 | " | 13.1 | |
| 11*** | " | 25.6 | |
| 12 | " | 32.5 | |
| 13 | " | 23.9 | |
| 14 | fertile | 63.8 | |
| 15 | " | 22.1 | |
| 16 | " | 65.9 | |
| 17 | " | 41.2 | LHM + 1 |
| 18 | " | 7.6 | |
| 19 | " | 35.3 | |
| 20 | postfertile | 33.7 | |
| 21 | " | | |
| 22 | " | | |
| 23 | " | | |
| 24 | " | | |
| 25 | " | | |
| 26 | " | | |
| 27 | " | | |
| 28 | " | | |
| 29 | " | | |
| 30 | " | | |

Days in advance of actual ovulation: 6
Chosen testing commencement day for next cycle: 11 (no change)

| CYCLE B 4 | | | |
|---|---|---|---|
| Day | Phase | E3G value | Actual Ovulation |
| 1 | infertile | 17.7 | |
| 2 | " | 12.2 | |
| 3 | " | 7.2 | |
| 4 | " | 6.2 | |
| 5 | " | 13.9 | |
| 6 | " | 12.9 | |
| 7 | " | 12.7 | |
| 8 | " | 9.3 | |
| 9 | " | 16.5 | |
| 10 | " | 17.7 | |
| 11*** | " | 26.0 | |
| 12 | " | 38.3 | |
| 13 | fertile | 70.6 | |
| 14 | " | 74.6 | |
| 15 | " | 70.6 | |
| 16 | " | 49.7 | LHM + 1 |
| 17 | " | 23.5 | |
| 18 | " | 29.8 | |
| 19 | postfertile | 44.4 | |
| 20 | " | 32.7 | |
| 21 | " | | |
| 22 | " | | |
| 23 | " | | |
| 24 | " | | |
| 25 | " | | |
| 26 | " | | |
| 27 | " | | |
| 28 | " | | |
| 29 | " | | |
| 30 | " | | |

Days in advance of actual ovulation: 5
Chosen testing commencement day for next cycle: 10

| CYCLE B 5 | | | |
|---|---|---|---|
| Day | Phase | E3G value | Actual Ovulation |
| 1 | infertile | 33.9 | |
| 2 | " | 27.3 | |
| 3 | " | 20.2 | |
| 4 | " | 7.0 | |
| 5 | " | 12.7 | |
| 6 | " | 7.2 | |
| 7 | " | 14.5 | |
| 8 | " | 14.8 | |
| 9 | " | 10.8 | |
| 10*** | " | 8.7 | |
| 11 | " | 14.1 | |
| 12 | " | 17.4 | |
| 13 | " | 41.3 | |
| 14 | " | 57.5 | |
| 15 | fertile | 42.0 | |
| 16 | " | 55.4 | |
| 17 | " | 60.1 | |
| 18 | " | 39.4 | LHM + 1 |
| 19 | " | 24.7 | |
| 20 | " | 10.5 | |
| 21 | postfertile | | |
| 22 | " | | |
| 23 | " | | |
| 24 | " | | |
| 25 | " | | |
| 26 | " | | |
| 27 | " | | |
| 28 | " | | |
| 29 | " | | |
| 30 | " | | |

Days in advance of actual ovulation: 8
Chosen testing commencement day for next cycle: 10 (no change)

## Claims

1. A method of monitoring the status of a current ovulation cycle of an individual mammalian female subject, involving repeated testing of the body fluid concentration of at least one analyte of significance in relation to the status of the ovulation cycle during at least the pre-ovulation phase of the current ovulation cycle of the individual subject, wherein testing for said analyte concentration during the current ovulation cycle is commenced a plurality of days following the onset of menses but at least 2 numerical days in advance of the earliest numerical day on which actual ovulation has occurred in one or more previous ovulation cycles in the same individual subject.

2. A method according to claim 1, wherein testing is commenced at least 3 numerical days in advance of the earliest numerical day on which actual ovulation has occurred in one or more previous ovulation cycles in the same individual subject.

3. A method according to claim 1, wherein testing is commenced at least 4 numerical days in advance of the earliest numerical day on which actual ovulation has occurred in one or more previous ovulation cycles in the same individual subject.

4. A method according to any one of the preceding claims, wherein testing is commenced at least 5 numerical days following the onset of menses.

5. A method according to claim 1, wherein the testing is commenced on a numerical day in advance of the earliest numerical day on which actual ovulation has occurred in one or more previous ovulation cycles in the same individual subject, said testing commencement day being calculated according to the following relationship:
| Earliest previous ovulation day | Testing commencement day |
|---|---|
| 8 - 10 | -4 |
| 11 - 14 | -5 |
| 15 - 18 | -6 |
| 19 - 23 | -7 |
| 24 - 28 | -8 |
| 29+ | -9 |

6. A method according to any one of the preceding claims, wherein the body fluid is urine.

7. A method according to any one of the preceding claims, wherein the analyte is estradiol or a metabolite thereof, such as E3G.

8. A method according to any one of the preceding claims, wherein 2 or more analytes are tested to provide comparative data, such as the concentration ratio of two analytes tested simultaneously.

9. A method according to any one of the preceding claims, wherein the earliest actual ovulation day is derived from data collected during at least 3 consecutive previous cycles.

10. A method according to any one of the preceding claims, wherein the earliest actual ovulation day is derived from data collected during at least 5 consecutive previous cycles.

11. A method according to any one of the previous claims, wherein the earliest ovulation day is derived from data obtained during at least the immediately preceding cycle.

12. A method according to claim 11, wherein the earliest ovulation day is derived from data obtained from a rolling reference base consisting of a fixed number of consecutive cycles immediately preceding the current cycle.

13. A method according to claim 12, wherein the rolling reference base consists of the immediately preceding 4 to 10 cycles.

14. A method according to claim 12, wherein the rolling reference base consists of the immediately preceding 5 or 6 cycles.

15. A method according to any one of the preceding claims, wherein actual ovulation day is determined by detecting the peak concentration of urinary LH.

16. A method according to any one of claims 1 to 14, wherein actual ovulation is determined by detecting the urinary LH surge.

17. A method according to any one of the preceding claims, wherein the subject is human.

18. A test kit for conducting a method according to any one of the preceding claims, comprising one or more testing devices for determining the concentration (in relative or absolute terms) of said at least one analyte in said body fluid, together with means enabling a user to derive a precise testing commencement day from knowledge of the numerical day on which actual ovulation occurred during at least one previous ovulation cycle.

19. A test kit according to claim 18, wherein said enabling means is electronic.

## Patentansprüche

1. Verfahren zum Überwachen des Status eines laufenden Ovulationszyklus eines weiblichen Untersuchungsindividuums der Säugetierklasse, bei dem die Konzentration in einer Körperflüssigkeit mindestens einer Nachweissubstanz mit Aussagekraft hinsichtlich des Status des Ovulationszyklus zumindest während der Präovulationsphase des laufenden Ovulationszyklus des Untersuchungsindividuums wiederholt getestet wird, wobei das Testen auf die Konzentration der Nachweissubstanz während des laufenden Ovulationszyklus eine Mehrzahl von Tagen nach dem Menstruationsbeginn, aber mindestens zwei numerische Tage vor dem frühesten numerischen Tag begonnen wird, an dem die tatsächliche Ovulation in einem oder mehreren früheren Ovulationszyklen des selben Untersuchungsindividuums aufgetreten ist.

2. Verfahren nach Anspruch 1, bei dem das Testen mindestens drei numerische Tage vor dem frühesten numerischen Tag begonnen wird, an dem die tatsächliche Ovulation in einem oder mehreren früheren Ovulationszyklen des selben Untersuchungsindividuums aufgetreten ist.

3. Verfahren nach Anspruch 1, bei dem das Testen mindestens vier numerische Tage vor dem frühesten numerischen Tag begonnen wird, an dem die tatsächliche Ovulation in einem oder mehreren früheren Ovulationszyklen des selben Untersuchungsindividuums aufgetreten ist.

4. Verfahren nach einen der vorstehenden Ansprüche, bei dem das Testen mindestens fünf numerische Tage nach dem Menstruationsbeginn begonnen wird.

5. Verfahren nach Anspruch 1, bei dem das Testen an einem numerischen Tag vor dem frühesten numerischen Tag begonnen wird, an dem die tatsächliche Ovulation in einem oder mehreren früheren Ovulationszyklen des selben Untersuchungsindividuums aufgetreten ist, wobei der Tag des Testbeginns nach der folgenden Beziehung berechnet wird:
| frühester früherer Ovulationstag | Tag des Testbeginns |
|---|---|
| 8 - 10 | -4 |
| 11 - 14 | -5 |
| 15 - 18 | -6 |
| 19 - 23 | -7 |
| 24 - 28 | -8 |
| 29+ | -9 |

6. Verfahren nach einem der vorstehenden Ansprüche, bei dem die Körperflüssigkeit Urin ist.

7. Verfahren nach einem der vorstehenden Ansprüche, bei dem die Nachweissubstanz Estradiol oder ein Metabolit hiervon, wie E3G, ist.

8. Verfahren nach einem der vorstehenden Ansprüche, bei dem zwei oder mehrere Nachweissubstanzen zur Bereitstellung von Vergleichsdaten, z.B. dem Konzentrationsverhältnis der zwei gleichzeitig getesteten Nachweissubstanzen, getestet werden.

9. Verfahren nach einem der vorstehenden Ansprüche, bei dem der früheste tatsächliche Ovulationstag aus Daten gewonnen wird, die während mindestens dreier aufeinanderfolgender früherer Zyklen gesammelt worden sind.

10. Verfahren nach einem der vorstehenden Ansprüche, bei dem der früheste tatsächliche Ovulationstag aus Daten gewonnen wird, die während mindestens fünf aufeinanderfolgender früherer Zyklen gesammelt worden sind.

11. Verfahren nach einem der vorstehenden Ansprüchen, bei dem der früheste Ovulationstag aus Daten gewonnen wird, die während zumindest des unmittelbar vorhergehenden Zyklus erhalten worden sind.

12. Verfahren nach Anspruch 11, bei dem der früheste Ovulationstag aus Daten gewonnen wird, die aus einer rollenden Referenzbasis erhalten sind, welche aus einer feststehenden Zahl von dem laufenden Zyklus unmittelbar vorhergehenden aufeinanderfolgenden Zyklen besteht.

13. Verfahren nach Anspruch 12, bei dem die rollende Referenzbasis aus den unmittelbar vorhergehenden 4 bis 10 Zyklen besteht.

14. Verfahren nach Anspruch 12, bei dem die rollende Referenzbasis aus den unmittelbar vorhergehenden 5 oder 6 Zyklen besteht.

15. Verfahren nach einem der vorstehenden Anspüche, bei dem der tatsächliche Ovulationstag durch Nachweis der Maximalkonzentration von LH im Urin bestimmt wird.

16. Verfahren nach einem der Ansprüche 1 bis 14, bei dem die tatsächliche Ovulation durch Nachweis des LH-Stoßes im Urin bestimmt wird.

17. Verfahren nach einem der vorstehenden Ansprüche, bei dem das Untersuchungsindividuum ein Mensch ist.

18. Testkit zur Durchführung eines Verfahrens nach einem der vorstehenden Ansprüche, umfassend eine oder mehrere Testvorrichtungen zur Bestimmung der Konzentration (relativ oder absolut) der mindestens einen Nachweissubstanz in der Körperflüssigkeit zusammen mit einer Einrichtung, die der Benutzerin gestattet, einen genauen Tag des Testbeginns aus der Kenntnis des numerischen Tages abzuleiten, an dem die tatsächliche Ovulation während mindestens eines früheren Ovulationszyklus stattgefunden hat.

19. Testkit nach Anspruch 18, bei dem die Vorrichtung eine elektronische Vorrichtung ist.

## Revendications

1. Procédé de contrôle de l'état d'un cycle d'ovulation en cours d'un sujet mammifère de sexe féminin, dans lequel on teste de façon répétée la concentration dans un liquide corporel d'au moins un analyte significatif par rapport à l'état du cycle d'ovulation au cours d'au moins la phase de préovulation du cycle d'ovulation en cours du sujet individuel, où les tests de concentration dudit analyte pendant le cycle d'ovulation en cours sont commencés plusieurs jours après l'apparition des règles mais au moins 2 jours calendaires avant le premier jour calendaire auquel l'ovulation effective s'est produite dans un ou plusieurs cycles d'ovulation antérieurs chez le même individu.

2. Procédé selon la revendication 1, dans lequel le test est commencé au moins 3 jours calendaire avant le premier jour calendaire auquel une ovulation effective s'est produite dans un ou plusieurs cycles d'ovulation antérieurs chez le même individu.

3. Procédé selon la revendication 1, dans lequel le test est commencé au moins 4 jours calendaires avant le premier jour calendaire auquel une ovulation effective s'est produite dans un ou plusieurs cycles d'ovulation antérieurs chez le même individu.

4. Procédé selon l'une quelconque des revendications précédentes, dans lequel on commence le test au moins 5 jours calendaires après l'apparition des règles.

5. Procédé selon la revendication 1, dans lequel le test commence un jour calendaire avant le premier jour calendaire auquel une ovulation effective s'est produite dans un ou plusieurs cycles d'ovulation antérieurs chez le même individu, ledit jour de commencement de test étant calculé selon la relation suivante:
| Premier jour d'ovulation antérieure | Jour de début des tests |
|---|---|
| 8 - 10 | - 4 |
| 11 - 14 | - 5 |
| 15 - 18 | - 6 |
| 19 - 23 | - 7 |
| 24 - 28 | - 8 |
| 29+ | - 9 |

6. Procédé selon l'une quelconque des revendications précédentes, dans lequel le liquide corporel est l'urine.

7. Procédé selon l'une quelconque des revendications précédentes, dans lequel l'analyte est l'oestradiol ou un de ses métabolites, comme l'E3G.

8. Procédé selon l'une quelconque des revendications précédentes, dans lequel on teste 2 ou plusieurs analytes pour fournir des données comparatives, comme le rapport de concentration de deux analytes testés simultanément.

9. Procédé selon l'une quelconque des revendications précédentes, dans lequel le premier jour d'ovulation effective est déduit de données recueillies au cours d'au moins 3 cycles antérieurs consécutifs.

10. Procédé selon l'une quelconque des revendications précédentes, dans lequel le premier jour d'ovulation effective est déduit de données recueillies pendant au moins 5 cycles antérieurs consécutifs.

11. Procédé selon l'une quelconque des revendications antérieures, dans lequel le premier jour d'ovulation est déduit de données obtenues pendant au moins le cycle immédiatement précédent.

12. Procédé selon la revendication 11, dans lequel le premier jour d'ovulation est déduit de données obtenues à partir d'une base de référence roulante constituée d'un nombre fixe de cycles consécutifs précédant immédiatement le cycle en cours.

13. Procédé selon la revendication 12, dans lequel la base de référence roulante se compose des 4 à 10 cycles immédiatement précédents.

14. Procédé selon la revendication 12, dans lequel la base de référence roulante se compose des 5 ou 6 cycles immédiatement précédents.

15. Procédé selon l'une quelconque des revendications précédentes, dans lequel on détermine le jour d'ovulation effective en détectant le pic de concentration de LH urinaire.

16. Procédé selon l'une quelconque des revendications 1 à 14, dans lequel on détermine l'ovulation effective en détectant la montée rapide de LH urinaire.

17. Procédé selon l'une quelconque des revendications précédentes, dans lequel le sujet est humain.

18. Nécessaire de test pour réaliser un procédé selon l'une quelconque des revendications précédentes, comprenant un ou plusieurs dispositifs de test pour déterminer la concentration (en termes relatifs ou absolus) dudit analyte, au moins un étant présent, dans ledit liquide corporel, avec un moyen permettant à une utilisatrice de déduire un jour précis de début de test à partir de la connaissance du jour calendaire auquel une ovulation effective s'est produite pendant au moins un cycle d'ovulation antérieur.

19. Nécessaire de test selon la revendication 18, dans lequel ledit moyen habilitant est électronique.
